# EUROPEAN PATENT SPECIFICATION

(11) **EP 3 444 337 B1**
(45) Date of publication and mention of the grant of the patent: **18.12.2024**
(21) Application number: 18189275.3
(22) Date of filing: 16.08.2018
(51) Int. Cl.: C12N 9/02, C11D 3/386, C12N 9/08

(54) **METHOD OF CLEANING**
REINIGUNGSVERFAHREN
PROCÉDÉ D'IMPRESSION

(30) Priority: 18.08.2017 EP 17186888
(43) Date of publication of application: 20.02.2019
(73) Proprietor: The Procter & Gamble Company, Cincinnati, OH 45202 (US)
(72) Inventor: LANT, Neil Joseph, Newcastle upon Tyne, NE12 9TS (GB); PATTERSON, Steven George, Newcastle upon Tyne, NE12 9TS (GB); VASQUEZ VALDIVIESO, Montserrat Guadalupe, Newcastle upon Tyne, NE12 9TS (GB)
(74) Representative: P&G Patent Belgium UK

(56) References cited:
- EP-B1- 2 350 250
- WO-A1-2007/036235
- WO-A1-2014/067933
- WO-A2-2007/045251
- US-A1- 2016 312 157

## Description

### TECHNICAL FIELD

The present invention is in the field of cleaning. It relates to a method of cleaning, in particular a method of cleaning a surface with a wash liquor in the presence of an oxidoreductase enzyme immobilized onto a supporting substrate. The invention also relates to the use of an oxidoreductase enzyme immobilized onto a supporting substrate to reduce malodour and dye transfer in a cleaning process.

### BACKGROUND OF THE INVENTION

When cleaning a surface by immersion in a wash liquor, dirt goes from the surface to be cleaned to the liquor. Dirt encompasses stains, soils, malodours, bacteria, etc. Dirt can be redeposited onto the surface being cleaned. There can also be transfer of colour from the surface being cleaned to the liquor. Colour bleeding can occur during the cleaning of a surface with a wash liquor. During the cleaning process dyes can migrate from the surface to the wash liquor. These dyes can be deposited onto other surfaces immersed in the wash liquor impairing on the appearance of the surface, similarly colours coming from stains can also be deposited onto the surface being cleaned. This can be more apparent in the case of laundry loads containing white fabrics. The white fabrics tend to become greyish when washed in the presence of fabrics that are not completely white. Dyes in the wash liquor can also contribute to colour deterioration of coloured fabrics. Soils, stains, bacteria, malodours removed from the fabrics can also being re-deposited on the fabrics in detriment of the cleaning process.

In the case of mixed laundry loads, i.e. loads containing coloured and white fabrics bleach cannot always be used because it could alter the colours of coloured fabrics. This can also be the case when cleaning patterned hard-surfaces.

Cleaning products being sold in the market can comprise enzymes. The enzymes usually found in laundry detergents are amylase, cellulase, protease and/or lipase. These enzymes are used in detergents as cleaning and fabric care agents. In order to remove stains, these enzymes typically first need to deposit onto the stains. Amylase, cellulase, protease and lipase have been immobilized on various substrates. For example, US 2013/0316430 A1 describes the immobilization of amylase, cellulase, protease and lipase on a PVC surface, in particular onto a plastic bucket and a brush for their application in cloth washing. WO 2014/006424 A1 is directed to a cleaning formulation comprising a multiplicity of solid cleaning particles, wherein said solid cleaning particles comprise polymeric particles and at least one cleaning agent, wherein said at least one cleaning agent is immobilised on the surface of said polymeric particles. Enzymes are among the cleaning agents recited in `424. In the case of `430 and `424 cleaning of fabrics seems to work by slowly releasing the enzymes into the wash liquor to access the fabrics.

Cleaning compositions comprising oxidoreductases are known, for example WO 2010/27755 A1 and WO 2016/37992 A1, however the oxidoreductases could attack the dyes on the fabrics.

EP2350250B1 describes methods, compositions, systems, and kits that include an enzyme/substrate co-delivery system. The liquid delivery system includes at least one enzyme encapsulated in a water-soluble polymeric matrix and a substrate for the enzyme in a carrier liquid in which the polymeric matrix is insoluble. When water is added, the polymeric matrix is solubilized and enzyme is released from the matrix, permitting catalytic action upon the substrate.

WO2007/036235A1 describes the immobilization of enzymes by adsorbing enzymes, a polyfunctional amine and a cross-linking agent onto a particulate porous carrier in a mixer apparatus or in a fluid bed apparatus.

WO2007/045251A2 describes a composition comprising a coupled enzyme system for the production of hydrogen peroxide by the coupling of a first enzyme system capable of hydrogen peroxide generation, to a second enzyme system which utilizes the non hydrogen peroxide product of the first enzyme system, and optionally is capable of generating further hydrogen peroxide.

US2016/312157A1 describes a detergent pouch, formed by an enzymatic water-soluble film, comprising a detergent; wherein the water-soluble film contains enzyme particles.

WO2014/067933A1 describes the use of insoluble enzyme preparations comprising an enzyme which is immobilized on a solid carrier having an average particle size of from about 0.1 to 500 µm as additives in consumer near applications.

The object of the present invention is to provide improved cleaning and at the same time protect the colour of surfaces. In particular, to provide cleaning while caring for the colours of coloured fabrics and prevent the greying of white fabrics in mixed loads as well as preventing malodours, and soil re-deposition.

### SUMMARY OF THE INVENTION

The invention is as defined by the claims.

According to the first aspect of the invention, there is provided a method for cleaning a surface. The method comprises the step of contacting the surface with a wash liquor, the wash liquor comprising a cleaning composition and an oxidoreductase enzyme immobilised on a supporting substrate by means of a chemical bond. Preferably the oxidoreductase enzyme is selected from the group consisting of laccase, peroxidase and a mixture thereof.

A "supporting substrate" within the meaning of the invention is any substrate capable of having an oxidoreductase enzyme immobilised on its surface.

An "oxidoreductase enzyme" is an enzyme that catalyzes the transfer of electrons from one molecule to another. The electron transfer can contribute to the decolourization of dyes and can help to avoid soil re-deposition and malodour. The object of this invention is to improve cleaning and this is achieved by promoting dyes decolourization and reducing soil re-deposition and malodour in the wash liquor and not on the surfaces being cleaned. The method can also prevent bacterial growth in the wash liquor and the surfaces being cleaned. This is achieved by immobilizing the oxidoreductase enzyme. Thus, the transfer of electrons would take place in the wash liquor, wherein the cleaning agent is located, and not on the surface to be cleaned, this would result in a cleaner wash liquor that would be translated into cleaner surfaces without exposing the surface to the chemical aggression that peroxidases can present.

The cleaning composition of the method of the invention can comprise a mediator. A "mediator" within the meaning of the invention is a small redox molecule that acts as an electron carrier between the supporting substrate to be oxidised and an oxidoreductase enzyme. Preferably, the mediator is a laccase mediator, in the case in which the oxidoreductase enzyme is a laccase or peroxidase mediator, in the case in which the oxidoreductase enzyme is a peroxidase . Once the mediator is oxidised, by giving one or several of its electrons to the oxidoreductase enzyme, it will oxidise dyes, soils, malodour, bacteria, etc, thereby cleaning the wash liquor and resulting in better cleaning. Mediators interact with the oxidoreductase enzyme to ensure the flow of electrons in the case of strongly different redox potentials between the oxidizing enzymes and supporting substrate upon which the enzyme will act.

Colour bleed can occur when fabrics, or any other surfaces, get wet and dye leaches out of the fibers. This commonly occurs in the washing machine and can result in colour transfer between items in the load.

There are two different ways to attack a dye, chemically, to remove its colour. One is by *oxidation,* in which electrons are removed, while the other is by *reduction,* in which electrons are added.

Chromophores cause colours by reflecting a certain portion of the visible spectrum of light. For example, a blue fabric contains chromophores that reflect blue light that our eyes see as the colour blue.

An oxidizing agent works by breaking the chemical bonds of a chromophore (part of a molecule that has colour). This changes the molecule so that it either has no colour or else reflects colour outside the visible spectrum.

A reducing agent works by changing the double bonds of a chromophore into single bonds. This alters the optical properties of the molecule, making it colourless.

The oxidoreductase enzyme in the method of the present invention is immobilized on a supporting substrate. The immobilization of the oxidoreductase enzyme makes the oxidation process to take place where or in close proximity to where the oxidoreductase enzyme is located rather than on the fabrics. As discussed before this results in better cleaning while caring for the cleaned surfaces. This differs from a traditional cleaning process where the enzymatic activity and bleaching take place on the soils deposited on the surfaces to be cleaned.

The method of the invention also comprises the step of contacting the surface with a wash liquor, the wash liquor comprising a cleaning composition and an oxidoreductase enzyme immobilised on a supporting substrate, and the oxidoreductase enzyme immobilised on the supporting substrate is re-used in a second wash step.

The oxidoreductase enzyme is immobilised on the substrate by means of chemical bond. The supporting substrate can be selected from the group consisting of fabrics, non-woven materials, plastics, and inorganic particles. In particular, supporting substrates in the form of a tri-dimensional hollow body that favours the flow of wash liquor through it are preferred herein. Plastic supporting substrates in the form of a tri-dimensional hollow body are prefer for use herein, more preferably when the oxidoreductase enzyme is immobilised on the inside of the hollow body. Also preferred are inorganic particles having a large surface area such as zeolites.

Preferably the cleaning composition of the method of the invention comprises a mediator, preferably selected from the group consisting of organic-based mediator, transition metal coordination complex mediator and a mixture thereof.

Also disclosed is the use of an oxidoreductase enzyme immobilized onto a supporting substrate to improve cleaning by reducing malodour and dye transfer in a cleaning process, preferably in a laundry process, more preferably the use of an oxidoreductase enzyme immobilized onto a substrate to reduce malodour and dye transfer in a laundry process when the laundry process involves a load of mixed colour fabrics in an automatic washing machine.

In a preferred method, following the first wash step in which the surface is cleaned by contacting the surface with the (first) wash liquor, the supporting substrate and oxidoreductase enzyme are separated from the (first) wash liquor and re-used in a second wash step for cleaning a second surface. In the second wash step the second surface is contacted with a second wash liquor, said second wash liquor comprising a cleaning composition and said oxidoreductase enzyme immobilized on said supporting substrate from the first wash step. The oxidoreductase enzyme can be separated from the first wash step for re-use either by removing the supporting substrate from the first wash liquor, or by removing the first wash liquor from the supporting substrate.

The elements of the method of the invention described in connection with the first aspect of the invention apply mutatis mutandis to the further aspects of the invention.

### DETAILED DESCRIPTION OF THE INVENTION

The present invention envisages a method for cleaning a surface comprising contacting the surface with a wash liquor, the wash liquor comprises a cleaning composition and an oxidoreductase enzyme, the oxidoreductase enzyme is immobilised on a supporting substrate. It also envisages the use of an oxidoreductase enzyme immobilized on a supporting substrate to reduce malodour, dye transfer in a cleaning process. The immobilized oxidoreductase enzyme is especially suited to be used in the laundry when the laundry load contains mixed colour fabrics.

As used herein, articles, for example, "a" and "an" when used in a claim, are understood to mean one or more of what is claimed or described.

### Immobilisation

Immobilisation of the oxidoreductase enzyme on the substrate is achieved by means of a chemical bond. Immobilisation can be achieved via chemical means including covalent, ionic, hydrogen, polar bonds.

Immobilisation of the oxidoreductase enzyme is preferably achieved by treating the supporting substrate with at least one activating agent in order to modify the chemical properties at the surfaces of the supporting substrate in order that the modified supporting substrate may subsequently be treated with at least one oxidoreductase enzyme in order to facilitate immobilization of the oxidoreductase enzyme.

The activated supporting substrate can then be further treated with a linking agent which facilitates attachment of the oxidoreductase enzyme by means of a chemical bond, preferably a covalent bond.

Activation of the surface may also be achieved by the use of physical agents, such as heat or electromagnetic radiation, e.g. ultra-violet radiation or microwave radiation prior to reaction with a linking agent.

Suitable linking agents may include glutaraldehyde, or may be selected from, for example, typical crosslinking agents such as dimethyl adipimidate, dimethyl suberimidate, pentafluorophenyl ester, hydroxymethyl phosphine, imidoesters and N-hydroxysuccinimide esters.

Other suitable linking agents include, for example:
N-Hydroxysuccinimide (NHS) and N-(3-Dimethylaminopropyl)-N'-ethylcarbodiimide hydrochloride (EDC);
Acylimidazoles (e.g. Carbonyl Diimidazole (CDI) and N,N'-carbonylbis(3- methylimidazolium) triflate (CBMIT);
Phosphonium salts (e.g. benzotriazol-1-yl-oxy-tris-(dimethylamino)-phosphonium hexafluorophosphate (BOP);
Uronium salts (e.g. 0-((ethoxycarbonyl)cyanomethylene amino)-N,N,N',N'- tetramethyl-uronium tetrafluoroborate (TOTU); and
Mukaiyama's reagent (2-chloro-1-methylpyridinium iodide).

Alternatively, embodiments utilising activating agents may include the treatment of polymeric particles incorporating polar groups, including for example Nylon 6,6 or poly(ethylene terephthalate), initially with a polar group-containing material - such as, for example, gelatin, starch, cellulose, chitosan, chitan, carboxymethylcellulose, poly(vinylimidazoles), poly(acrylic acid), poly(methacrylic acid), poly(lactic acid), poly(maleic acid), poly(glycolic acid), poly(acrylonitrile), poly(vinylpyrrolidone), poly(dimethylaminoethyl methacrylate), poly(ethylene imine), poly(allylamine), poly(allylamine) hydrochloride, poly(ethylene glycol), polypropylene glycol), poly(acrylamide), polyvinyl alcohol), polyvinyl acetate), polyvinyl formamide), poly(vinylamine), amine-containing molecules (including biomolecules such as proteins), carboxylic acids such as maleic acid and itaconic acid, and carboxylic acid-containing polymers, as well as derivatives and copolymers of all the foregoing - wherein ionic interactions are formed between the polymer particles and a layer of the polar group-containing material, and subsequently with the oxidoreductase enzyme wherein further ionic interactions are established between the layer of polar group-containing material and the layer of oxidoreductase enzyme.

Optionally, said embodiments utilising at least one activating agent may comprise multiple treatments with the at least one activating agent and/or multiple subsequent treatments or reactions with the at least one mediator. Said embodiments, which rely on ionic interactions, do not require the use of a linker.

### Supporting substrates

### Porous supporting substrates are preferred for use herein.

A variety of materials that can be used as supporting substrate for immobilization of the mediator include polymeric materials (plastics), including natural or synthetic or partially synthetic polymeric materials for example, cellulose, polystyrene, gelatin, agar, acrylate polymers such as poly(2-hydroxyethyl methacrylate), poly (methyl methacrylate-acrylic acid), polyacrylamide, acrylonitrile/acrylamide polymers, polyesters, alginates, poly (vinyl alcohol) PVA, polyurethane, homo or copolymers. These may be in any form, for example, the substrate may be in the form of a shaped or moulded article, sheet, film, woven or non-woven article, fibres, foam, gel, bead, spheres. Preferred examples include cellulose, polystyrene, alkylamine glass beads through covalent coupling, cation exchange resin, photographic gelatin, plastic supports, agar gel, acrylonitrile/acrylamide membranes, poly(2-hydroxyethyl methacrylate) microspheres, poly (methyl methacrylate-acrylic acid) microspheres, polyacrylamide gel, glass beads, sodium alginate beads, superporous celbeads, polyster surface free and affixed alkyl and arylamine glass beads, alginate gel beads, cyclic carbonate bearing hybrid materials, cellulose fibre materials and cellulose-coated magnetite (CCM) nanoparticles.

Other preferred materials suitable as supporting substrate for immobilization of the mediator include polyurethane foam, tri(4-formyl phenoxy) cyanurate, polyacrylamide-acrylic gel, acrylamide grafted acrylonitrile copolymer (PAN), chemically modified pumic particles, nanofibrous poly (vinyl alcohol) PVA, passive epoxy acrylate films modified by magnetic filtered plasma stream, silicate clay mineral, modified polyvinyl alcohol coated chitosan beads, loofa sponge, liposomes, brick dust via glutaraldehyde and silicon wafers of amino terminated surface.

Other suitable supporting substrates for immobilization of the mediator are particles, preferably selected from inorganic particles, however, some organic particles can also be used. A preferred supporting substrate herein is selected from the group consisting of a silica particle, a zeolite, an aluminum oxide, an organic polymer having either a carboxyl or an amino group, and a mixture thereof. These organic polymers are, preferably, selected from the group consisting of a polyacrylic acid, a polymaleic acid, a poly peptide, chitosan and a mixture thereof. Preferably, the supporting substrate has a median particle size (as measured as the diameter of the particle) of from about 1 nanometer to about 10 micrometers, more preferably, from about 1 nanometer to about 1 micrometer and even more preferably, the supporting substrate is selected from a silica having a particle size of from about 5 nanometers to about 1 micrometer. The median particle size is measured by SEM (Scanning Electron Microscope). A highly preferred silica is SiOx (MN1P, which is provided by Zhou Shan Ming Ri Nano Material Company (Zhejiang Province, China). Other preferred supporting substrates are described in PCT patent publication No. WO 90/04181 which is assigned to Nilsson, published on Apr. 19, 1990.

When an inorganic particle is selected as the supporting substrate, it must be modified by a linking molecule before being activated. Any compounds which can provide the substrate with either carboxyl and/or amino groups can be used as a linking molecule herein. A suitable linking molecule is a silane linking molecule, preferably the structure of the silane molecule is R₁-(CH₂)ₙ₁-Si(O(CH₂)ₙ₂CH₃)₃, wherein R₁ is selected from -COOH or -NH₂; n1 is from about 1 to about 16, preferably from about 3 to about 8; n2 is from about 0 to about 10, preferably from about 0 to about 4. A preferred linking molecule for use herein is 3-aminopropyltriethoxysilane (APS). The weight ratio of the linking molecule to the supporting substrate is preferably from about 0.001:1 to about 10:1, and more preferably from about 0.1:1 to about 5:1. Other linking molecules useful herein are described in U.S. Pat. No. 6,004,786 to Yamashita, et al., issued Dec. 21, 1999.

The linking molecule modifies the supporting substrate to connect the supporting substrate and the oxidoreductase enzyme. In some instances, it is preferred to add a functional group introducer together with the linking molecule to the supporting substrate. A preferred functional group introducer is a carboxylic group introducer or an amino group introducer, more preferably a carboxylic group introducer such as a carboxylic acid anhydride. It is conceivable that the linking molecule itself may sometimes work as the functional group introducer. For example, when selecting carboxylic silane as the linking molecule, an additional functional group introducer is not necessary.

The modification of the supporting substrate by the linking molecule or functional group introducer can be accomplished by mixing the supporting substrate with the linking molecule with functional group introducer into a common organic solvent such as toluene, and re-fluxing for from about 4 hours to about 7 hours, preferably about 6 hours. The refluxed mixture is extracted by filtration, washed with ethanol and dried at about 30° C to about 70° C, preferably from about 45° C to about 55° C, for 20 minutes. The mixture is preferably kept in the vacuum dry container until being applied to next step.

Preferred carboxylic acid anhydrides are selected from the group consisting of a succinic anhydride, a maleic anhydrides, or a mixture thereof. In order to link a carboxyl group onto the supporting substrate, the supporting substrate is usually dissolved in organic solvents, preferably, a mixture of pyridine and anhydrous diethylether, and is mixed with a carboxylic acid anhydride at 25° C, for 17 hours. After mixing, the mixture is extracted by filtration and washed with organic solvents, preferably, anhydrous diethylether is used.

After the supporting substrate has been modified, an activating molecule activates the supporting substrate to connect or entrap the oxidoreductase enzyme onto the supporting substrate. The activation can be performed by adding an activating molecule to the activated supporting substrate and stirring together for from about 30 minutes to about 60 minutes, at 4° C. A preferable activating molecule for use herein is a water soluble carbon diimide. More preferably, the water soluble carbon diimide is selected from the group consisting of ethyl-3-(3-dimethyaminopropyl)-carbon diimide hydrochloride (EDC), a succinimide, and a mixture thereof. The weight ratio of the activating molecule to the supporting substrate is preferably from about 0.01:1 to about 1:1, more preferably, from about 0.05: 1 to about 0.5:1. After the substrate is activated, the substrate is isolated by centrifuging the sample and decanting the supernatant.

A suitable way to immobilize oxidoreductase enzymes is by coating the supporting substrate with polyphenol. The coating can be formed on diverse material surfaces under mild aqueous conditions. Examples of polyphenols include tannic acid, pyrogallol, pyrogallol 2-aminoethane, dopamine, etc, tannic acid is preferred for use herein.

### Supporting substrate configuration

The substrate can have any configuration but it would preferably have a configuration that promotes the contact between the mediator and the wash liquor and avoid the contact with the surface to be cleaned. Preferably, the supporting substrate will be a shaped or moulded article such as a tri-dimensional hollow body and the mediator would be placed on the inside of the hollow body. Other preferred supporting substrates for use herein are particles in which the oxidoreductase enzyme has been immobilized in the internal surface of the particle. Zeolites are preferred for use herein. Non-woven supporting substrates are also preferred for use herein.

### Oxidoreductase Enzyme

The cleaning composition of the invention preferably comprises oxidoreductase enzymes from the enzyme classification E.C. 1.

Laccases are preferred oxidoreductase enzyme for use herein, in particular laccases belonging to the enzyme classification (EC 1.10.3.2) and any chatechol oxidase enzyme comprised by the enzyme classification (EC 1.10.3.1). Additionally any monophenol monooxygenase enzyme comprised by the enzyme classification (EC 1.14.99.1); any bilirubin oxidase enzyme comprised by the enzyme classification (EC 1.3.3.5). Other commercially available oxidoreductase enzymes include ascorbate oxidase, cellobiose dehydrogenase, glucose oxidase, hexose oxidase and sulfhydryl oxidase.

Preferred laccases of the present invention include:
a) variants of the wild-type laccase from *Myceliophthora thermophila* which has at least 70%, preferably at least 80% identity with the amino acid sequence SEQ ID NO:1.
b) variants of the wild-type laccase from *Bacillus licheniformis* which has at least 70%, preferably at least 80% identity with the amino acid sequence SEQ ID NO:2
c) variants of the wild-type laccase from *Streptomyces sviceus* which has at least 70%, preferably at least 80% identity with the amino acid sequence SEQ ID NO:3

Oxidoreductase enzyme within the context of this invention includes peroxidases. Peroxidases are well described as enzymes which can be used to catalyse the oxidation reaction of a supporting substrate with hydrogen peroxide. An enzyme exhibiting peroxidase activity may be any peroxidase enzyme comprised by the enzyme classification (EC 1.11.1.x), or any fragment derived therefrom, exhibiting peroxidase activity. Suitable peroxidases/oxidases include those of plant, bacterial or fungal origin. Chemically modified or protein engineered mutants are included. Examples of useful peroxidases include peroxidases from *Coprinus,* e.g. from *C*. *cinereus,* and variants thereof as those described in WO 9324618, WO 9510602, and WO 9815257.

Commercially available peroxidases include Guardzyme^{™} (Novo Nordisk A/S).

Preferred peroxidases of the present invention include:
a) variants from *Bjerkandera adusta* which has at least 65%, preferably at least 80% identity with the amino acid sequence SEQ ID NO:4
b) variants from *Bjerkandera adusta* which has at least 70%, preferably at least 80% identity with the amino acid sequence SEQ ID NO: 5
c) variants from *Bjerkandera adusta* which has at least 70%, preferably at least 80% identity with the amino acid sequence SEQ ID NO:6
d) variants from *Ganoderma applanatum* which has at least 70%, preferably at least 80% identity with the amino acid sequence SEQ ID NO:7

More preferred peroxidases of the present invention include dye decolorizing peroxidases (EC 1.11.1.19) including dye decolorizing peroxidase from *Thermobifida fusca* in which has at least 60% identity for amino acid sequence SEQ ID NO:8.

### Hydrogen peroxide generating enzyme

An enzyme that is capable of generating hydrogen peroxide when said enzyme interacts with a substrate. A hydrogen peroxide generating enzyme can be glucose oxidase, carbohydrate: acceptor oxidoreductase (CAOX), cellobiose dehydrogenase, cellobiose quinone oxidoreductase (CBQ), cellooligosaccharide oxidoreductase (COOX), glucooligosaccharide oxidoreductase (GOOX), malate oxidase, hexose oxidase, galactose oxidase, pyranose oxidase, L-sorbate oxidase, lytic polysaccharide monooxygenase, alcohol oxidase, polyvinyl-alcohol oxidase, xylitol oxidase, D-Mannitol oxidase, L-Gulonolactone, N-Acylhexosamine oxidase and D-Arabinono-1,4 -lactone oxidase.

When the oxidoreductase enzyme for use in the method of the invention is a peroxidase, the cleaning composition comprises from 0.01% to 5%, preferably from 0.1% to 2% by weight of the composition of a peroxygen source. The peroxygen source is selected from the group consisting of hydrogen peroxide, a hydrogen peroxide precursor, a hydrogen peroxide generating enzyme system, or a peroxycarboxylic acid or a salt thereof and a mixture thereof.

### Mediator

A mediator is a small redox molecule that acts as an electron carrier between the substrate to be oxidised and the oxidising enzyme. Once the mediator is oxidised, by giving one or several of its electrons to the oxidoreductase enzyme, it oxidises dyes, malodours, bacteria, soil, etc. The oxidoreductase enzyme then gains electrons from the substrate that is oxidised to return to its reduced state, making it available once again for oxidation by the oxidoreductase enzyme. Overall, the oxidoreductase enzyme-mediator system acts as a catalyst to oxidise substances in the wash liquor. In the present case, the immobilized mediators are activated by an oxidoreductase enzyme. The mediators according to the invention include the chemical structure: 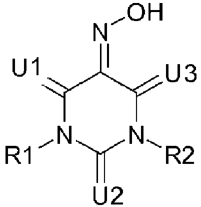 wherein U1 , U2 and U3 are identical or different, and are O, S or NOH; and R1 and R2 are identical or different, and are hydrogen, hydroxyl, formyl, carbamoyl or sulfono radical, ester or salt of the sulfono radical, sulfamoyl, nitro, nitroso, amino, cyano, phenyl, benzyl, CrC4-alkyl, Ci-C4-alkoxy, Ci-C4-carbonyl, carbonyl-Ci-C4-alkyl.

In an embodiment, U1, U2 and U3 are identical or different, and are O or S; and R1 and R2 are identical or different, and are hydrogen, hydroxyl, formyl, carbamoyl or sulfono radical, ester or salt of the sulfono radical, sulfamoyl, nitro, nitroso, amino, cyano, phenyl, benzyl, Ci-C4- alkyl, Ci-C4-alkoxy, Ci-C4-carbonyl, carbonyl-Ci-C4-alkyl.

In another embodiment, U1, U2 and U3 are O; and R1 and R2 are identical or different, and are hydrogen, hydroxyl, formyl, carbamoyl or sulfono radical, ester or salt of the sulfono radical, sulfamoyl, nitro, nitroso, amino, cyano, phenyl, benzyl, Ci-C4-alkyl, Ci-C4-alkoxy, Ci-C4-carbonyl, carbonyl-Ci-C4-alkyl.

In another embodiment, U1, U2 and U3 are identical or different, and are O, S or NOH; and R1 and R2 are identical or different, and are hydrogen, hydroxyl, methyl, ethyl, phenyl, benzyl, formyl, amino, cyano, nitroso, methoxy and/or ethoxy. In another embodiment, U1, U2 and U3 are identical or different, and are O or S; and R1 and R2 are identical or different, and are hydrogen, hydroxyl, methyl, ethyl, phenyl, benzyl, formyl, amino, cyano, nitroso, methoxy and/or ethoxy. In another embodiment, U1, U2 and U3 are O; and R1 and R2 are identical or different, and are hydrogen, hydroxyl, methyl, ethyl, phenyl, benzyl, formyl, amino, cyano, nitroso, methoxy and/or ethoxy.

Mediators could be 1 -methylvioluric acid, 1 ,3-dimethylvioluric acid, thiovioluric acid and violuric acid (alloxan-4,5-dioxime).

The mediator could also be alloxan-5-oxime (violuric acid) and/or its esters, ethers or salts.

Examples of enhancers and mediators are disclosed in EP 705327; WO 98/56899; EP677102; EP 781328; and EP 707637. If desired a distinction could be made by defining an oxidoreductase enzyme system (e.g. a laccase, or a peroxidase enzyme system) as the combination of the enzyme in question and its acceptor, and optionally also an enhancer and/or mediator for the enzyme in question.

Another mediator is hydroxyl benzoate and hydroxyl benzotriazole.

The mediator may be selected from the group consisting of aliphatic, cyclo- aliphatic, heterocyclic or aromatic compounds containing the moiety >N-OH. The mediator could include a compound of the general formula I: 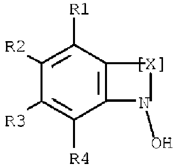 wherein R1 , R2, R3, R4 are individually selected from the group consisting of hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof, wherein the R1 , R2, R3, R4 may be substituted with R5, wherein R5 represents hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof; [X] represents a group selected from (-N=N-), (-N=CR6-)m, (-CR6=N-)m, (-CR7=CR8-)m, (-CR6=N- NR7-), (-N=N-CHR6-), (-N=CR6-N R7-), (-N=CR6-CH R7-), (-CR6=N-CHR7-), (-CR6=CR7-NR8-), and (-CR6=CR7-CH R8-), wherein R6, R7, and R8 independently of each other are selected from H, OH, NH2, COOH, S03H, Ci-6-alkyl, N02, CN , CI, Br, F, CH2OCH3, OCH3, and COOCH3; and m is 1 or 2.

The term "C1-n-alkyl" wherein n can be from 2 through 12, as used herein, represent a branched or straight alkyl group having from one to the specified number of carbon atoms. Typical Ci-6-alkyl groups include, but are not limited to, methyl, ethyl, n-propyl, iso-propyl, butyl, iso-butyl, sec-butyl, tert-butyl, pentyl, iso-pentyl, hexyl, iso-hexyl and the like.

The mediator could include a compound of the general formula II : 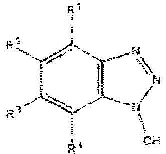 wherein R1, R2, R3, R4 are individually selected from the group consisting of hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof, wherein the R1 , R2, R3, R4 may be substituted with R5, wherein R5 represents hydrogen, halogen, hydroxy, formyl, carboxy and salts and esters thereof, amino, nitro, Ci-i2-alkyl, Ci-6-alkoxy, carbonyl(Ci-i2-alkyl), aryl, in particular phenyl, sulfo, aminosulfonyl, carbamoyl, phosphono, phosphonooxy, and salts and esters thereof.

The mediator may also be a salt or an ester of formula I or II .

The mediator may also be oxoderivatives and N-hydroxy derivatives of heterocyclic compounds and oximes of oxo- and formyl-derivatives of heterocyclic compounds, said heterocyclic compounds including five-membered nitrogen-containing heterocycles, in particular pyrrol, pyrazole and imidazole and their hydrogenated counterparts (e.g. pyrrolidine) as well as triazoles, such as 1 ,2,4-triazole; six-membered nitrogen-containing heterocycles, in particular mono-, di- and triazinanes (such as piperidine and piperazine), morpholine and their unsaturated counterparts (e.g. pyridine and pyrimidine); and condensed heterocycles containing the above heterocycles as substructures, e.g. indole, benzothiazole, quinoline and benzoazepine.

Examples of mediators from these classes of compounds are pyridine aldoximes; N-hydroxypyrrolidinediones such as N-hydroxysuccinimide and N- hydroxyphthalimide; 3,4-dihydro-3-hydroxybenzo[1 ,2,3]triazine-4-one; formaldoxime trimer (N,N',N"-trihydroxy-1 ,3,5-triazinane); and violuric acid (1 ,3-diazinane-2,4,5,6-tetrone-5-oxime).

Other mediators which may be applied in the invention include oximes of oxo- and formyl-derivatives of aromatic compounds, such as benzoquinone dioxime and salicylaldoxime (2-hydroxybenzaldehyde oxime), and N-hydroxyamides and N-hydroxyanilides, such as N-hydroxyacetanilide.

Mediators could also be selected from the group consisting of 1 - hydroxybenzotriazole; 1 - hydroxybenzotriazole hydrate; 1 -hydroxybenzotriazole sodium salt; 1 - hydroxybenzotriazole potassium salt; 1 -hydroxybenzotriazole lithium salt; 1 - hydroxybenzotriazole ammonium salt; 1 -hydroxybenzotriazole calcium salt; 1 - hydroxybenzotriazole magnesium salt; and 1 - hydroxybenzotriazole-6-sulphonic acid.

All the specifications of N-hydroxy compounds above are understood to include tautomeric forms such as N-oxides whenever relevant.

Another group of mediators comprises a -CO-NOH- group and has the general formula III: 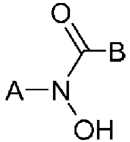 in which A is: 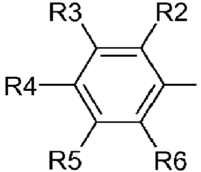 and B is the same as A; or B is H or Ci-i2-alkyl, said alkyl may contain hydroxy, ester or ether groups (e.g. wherein the ether oxygen is directly attached to A-N(OH)C=0-, thus including N-hydroxy carbamic acid ester derivatives), and R2, R3, R4, R5 and R6 independently of each other are H, OH, N H2, COOH , S03H , d-8-alkyl, acyl, N02, CN, CI, Br, F, CF3, NOH-CO-phenyl, CO-NOH-phenyl, Ci-6-CO-NOH-A, CO-NOH-A, COR12, phenyl-CO-NOH-A, OR7, NR8R9, COOR10, or NOH-CO-R1 1 , wherein R7, R8, R9, R10, R1 1 and R12 are C1-12-alkyl or acyl. R2, R3, R4, R5 and R6 of A are preferably H, OH, NH2, COOH, S03H, C1-3-alkyl, acyl, N02, CN, CI, Br, F, CF3, NOH-CO-phenyl, CO-NOH-phenyl, COR12, OR7, NR8R9, COOR10, or NOH-CO-R1 1 , wherein R7, R8 and R9 are d-3-alkyl or acyl, and R10, R1 1 and R12 are Ci-3-alkyl; more preferably R2, R3, R4, R5 and R6 of A are H, OH, NH2, COOH, S03H, CH3, acyl, N02, CN, CI, Br, F, CF3, CO-NOH-phenyl, COCH3, OR7, NR8R9, or COOCH3, wherein R7, R8 and R9 are CH3 or COCH3; even more preferably R2, R3, R4, R5 and R6 of A are H, OH, COOH, S03H, CH3, acyl, N02, CN, CI, Br, F, CO-NOH-phenyl, OCH3, COCH3, or COOCH3; and in particular R2, R3, R4, R5 and R6 of A are H, OH, COOH, S03H, CH3, N02, CN, CI, Br, CO-NOH-phenyl, or OCH3.

R2, R3, R4, R5 and R6 of B are preferably H, OH, NH2, COOH, S03H, C1-3-alkyl, acyl, N02, CN, CI, Br, F, CF3, NOH-CO-phenyl, CO-NOH-phenyl, COR12, OR7, NR8R9, COOR10, or NOH-CO-R1 1 , wherein R7, R8 and R9 are C1-3-alkyl or acyl, and R10, R1 1 and R12 are Ci-3-alkyi; more preferably R2, R3, R4, R5 and R6 of B are H, OH, NH2, COOH, S03H, CH3, acyl, N02, CN, CI, Br, F, CF3, CO-NOH-phenyl, COCH3, OR7, NR8R9, or COOCH3, wherein R7, R8 and R9 are CH3 or COCH3; even more preferably R2, R3, R4, R5 and R6 of B are H, OH, COOH, S03H, CH3, acyl, N02, CN, CI, Br, F, CO-NOH-phenyl, OCH3, COCH3, or COOCH3; and in particular R2, R3, R4, R5 and R6 of B are H, OH, COOH, S03H, CH3, N02, CN, CI, Br, CO-NOH-phenyl, or OCH3.

B is preferably H or Ci-3-alkyl, said alkyi may contain hydroxy, ester or ether groups; preferably said alkyi may contain ester or ether groups; more preferably said alkyi may contain ether groups. In an embodiment, A and B independently of each other are: 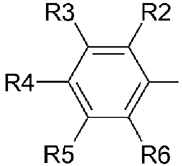 or B is H or Ci-3-alkyl, said alkyi may contain hydroxy, ester or ether groups (e.g. wherein the ether oxygen is directly attached to A-N(OH)C=0-, thus including N-hydroxy carbamic acid ester derivatives), and R2, R3, R4, R5 and R6 independently of each other are H, OH, NH2, COOH, S03H, Ci-3-alkyl, acyl, N02, CN, CI, Br, F, CF3, NOH-CO-phenyl, CO-NOH-phenyl, COR12, OR7, NR8R9, COOR10, or NOH-CO-R1 1 , wherein R7, R8 and R9 are C1-3-alkyl or acyl, and R10, R1 1 and R12 are C1-3-alkyl.

In another embodiment, A and B independently of each other are: 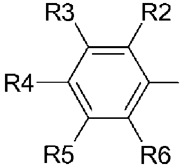 or B is H or Ci-3-alkyl, said alkyl may contain hydroxy or ether groups (e.g. wherein the ether oxygen is directly attached to A-N(OH)C=0-, thus including N-hydroxy carbamic acid ester derivatives), and R2, R3, R4, R5 and R6 independently of each other are H, OH, NH2, COOH, S03H, CH3, acyl, N02, CN, CI, Br, F, CF3, CO-NOH-phenyl, COCH3, OR7, NR8R9, or COOCH3, wherein R7, R8 and R9 are CH3 or COCH3.

In another embodiment, A and B independently of each other are: 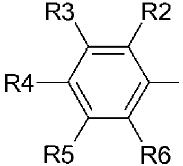 or B is H or Ci-3-alkyl, said alkyl may contain hydroxy or ether groups (e.g. wherein the ether oxygen is directly attached to A-N(OH)C=0-, thus including N-hydroxy carbamic acid ester derivatives), and R2, R3, R4, R5 and R6 independently of each other are H, OH, COOH, S03H, CH3, acyl, N02, CN , CI, Br, F, CO-NOH-phenyl, OCH3, COCH3, or COOCH3.

In another embodiment, A and B independently of each other are: 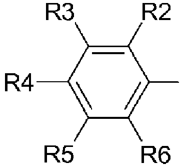 or B is Ci-3-alkyl, said alkyl may contain ether groups (e.g. wherein the ether oxygen is directly attached to A-N(OH)C=0-, thus including N-hydroxy carbamic acid ester derivatives), and R2, R3, R4, R5 and R6 independently of each other are H, OH, COOH, S03H, CH3, N02, CN, CI, Br, CO-NOH-phenyl, or OCH3.

The terms "Ci-n-alkyl" wherein n can be from 2 through 12, as used herein, represent a branched or straight alkyl group having from one to the specified number of carbon atoms. Typical Ci-6-alkyl groups include, but are not limited to, methyl, ethyl, n-propyl, iso-propyl, butyl, iso-butyl, sec-butyl, tert-butyl, pentyl, iso-pentyl, hexyl, iso-hexyl and the like.

The term "acyl" as used herein refers to a monovalent substituent comprising a Ci-6-alkyl group linked through a carbonyl group; such as e.g. acetyl, propionyl, butyryl, isobutyryl, pivaloyl, valeryl, and the like. In an embodiment, at least one of the substituents R2, R3, R4, R5 and R6 of A are H, preferably at least two of the substituents R2, R3, R4, R5 and R6 of A are H, more preferably at least three of the substituents R2, R3, R4, R5 and R6 of A are H, most preferably at least four of the substituents R2, R3, R4, R5 and R6 of A are H, in particular all of R2, R3, R4, R5 and R6 of A are H.

In another embodiment, at least one of the substituents R2, R3, R4, R5 and R6 of B are H, preferably at least two of the substituents R2, R3, R4, R5 and R6 of B are H, more preferably at least three of the substituents R2, R3, R4, R5 and R6 of B are H, most preferably at least four of the substituents R2, R3, R4, R5 and R6 of B are H, in particular all of R2, R3, R4, R5 and R6 of B are H.

In particular embodiments according to the invention, the mediator is selected from the group consisting of
4-nitrobenzoic acid-N-hydroxyanilide;
4-methoxybenzoic acid-N-hydroxyanilide;
N,N'-dihydroxy-N,N'-diphenylterephthalamide;
decanoic acid-N-hydroxyanilide;
N-hydroxy-4-cyanoacetanilide;
N-hydroxy-4-acetylacetanilide;
N-hydroxy-4-hydroxy acetanilide;
N-hydroxy-3-(N'-hydroxyacetamide)acetanilide;
4-cyanobenzoic acid-N-hydroxyanilide;
N-hydroxy-4-nitroacetanilide;
N-hydroxyacetanilide;
N-hydroxy-N-phenyl-carbamic acid isopropyl ester;
N-hydroxy-N-phenyl-carbamic acid methyl ester;
N-hydroxy-N-phenyl-carbamic acid phenyl ester;
N-hydroxy-N-phenyl-carbamic acid ethyl ester; and
N-hydroxy-N-(4-cyanophenyl)-carbamic acid methyl ester.

Another group of mediators is phenolic compounds (alkylsyringates) of the general formula IV: 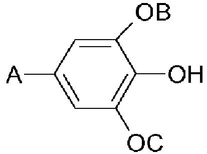 wherein the letter A in said formula denotes be a group such as -D, -CH=CH-D, -CH=CH-CH=CH-D, -CH=N-D, -N=N-D, or -N=CH-D, in which D is selected from the group consisting of - CO-E, -S02-E, -N-XY, and -NΓ-XYZ, in which E may be -H, -OH, -R, or -OR, and X and Y and Z may be identical or different and selected from -H and -R; R being a Ci-Ci6 alkyl, preferably a Ci-C8 alkyl, which alkyl may be saturated or unsaturated, branched or unbranched and optionally substituted with a carboxy, sulpho or amino group; and B and C may be the same or different and selected from CmH2m+i, where m = 1 , 2, 3, 4 or 5.

In the above mentioned general formula IV, A may be placed meta to the hydroxy group instead of being placed in the para-position as shown.

In particular embodiments of the invention, the mediator is selected from the group having the general formula V: 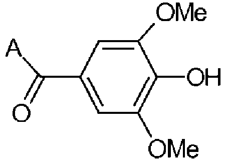 in which A is a group such as -H, -OH, -CH3, -OCH3, -0(CH2)nCH3, where n = 1 , 2, 3, 4, 5, 6, 7 or 8.

Yet another group of mediators are the compounds as described in general formula VI: 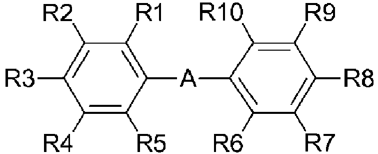 in which general formula A represents a single bond, or one of the following groups: (- CH2-), (-CH=CH-), (-NR1 1 -), (-CH=N-), (-N=N-), (-CH=N-N=CH-), or (>C=0);
and in which general formula the substituent groups R1 -R1 1, which may be identical or different, independently represents any of the following radicals: hydrogen, halogen, hydroxy, formyl, acetyl, carboxy and esters and salts hereof, carbamoyl, sulfo and esters and salts hereof, sulfamoyl, methoxy, nitro, amino, phenyl, Ci-8-alkyl; which carbamoyl, sulfamoyl, phenyl, and amino groups may furthermore be unsubstituted or substituted once or twice with a substituent group R12; and which Ci-8-alkyl group may be saturated or unsaturated, branched or unbranched, and may furthermore be unsubstituted or substituted with one or more substituent groups R12; which substituent group R12 represents any of the following radicals: hydrogen, halogen, hydroxy, formyl, acetyl, carboxy and esters and salts hereof, carbamoyl, sulfo and esters and salts hereof, sulfamoyl, methoxy, nitro, amino, phenyl, or Ci-8-alkyl; which carbamoyl, sulfamoyl, and amino groups may furthermore be unsubstituted or substituted once or twice with hydroxy or methyl;
and in which general formula R5 and R6 may together form a group -B-, in which B represents a single bond, one of the following groups (-CH2-), (-CH=CH-), (-CH=N-); or B represents sulfur, or oxygen.

In particular embodiments of the invention, the mediator is selected from the group having the general formula VII: 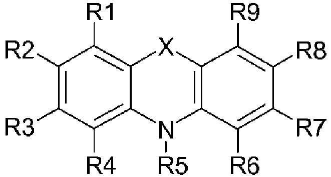 in which general formula X represents a single bond, oxygen, or sulphur;
and in which general formula the substituent groups R1 -R9, which may be identical or different, independently represents any of the following side groups: hydrogen, halogen, hydroxy, formyl, acetyl, carboxy and esters and salts hereof, carbamoyl, sulfo and esters and salts hereof, sulfamoyl, methoxy, nitro, amino, phenyl, Ci-8-alkyl;
which carbamoyl, sulfamoyl, phenyl, and amino groups may furthermore be unsubstituted or substituted once or twice with a substituent group R10; and which Ci-8-alkyl group may be saturated or unsaturated, branched or unbranched, and may furthermore be unsubstituted or substituted with one or more substituent groups R10;
which substituent group R10 represents any of the following radicals: hydrogen, halogen, hydroxy, formyl, acetyl, carboxy and esters and salts hereof, carbamoyl, sulfo and esters and salts hereof, sulfamoyl, methoxy, nitro, amino, phenyl, or Ci-8-alkyl; which carbamoyl, sulfamoyl, and amino groups may furthermore be unsubstituted or substituted once or twice with hydroxy or methyl.

Another mediator according to the invention is 2,2',6,6'-tetramethyl- piperidine-/V-ox l (TEMPO):

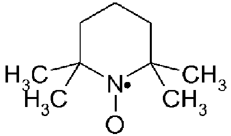

### Most preferred mediators

Most preferred organic based mediators are selected from the group consisting of: 2,2'-azinobis-(3-ethylbenzthiazoline-6-sulfonate), 1-hydroxybenzotriazole, violuric acid, N-hydroxyacetanilide, methyl syringate, acetosyringone, syringaldezine, butyl syringate, pentyl syringate, hexyl syringate, heptyl syringate, vanillyl alcohol, synapic acid and acetovanillone, and mixtures thereof, particularly preferred are methyl syringate, acetosyringone, syringaldezine, butyl syringate, pentyl syringate, hexyl syringate, heptyl syringate, vanillyl alcohol, synapic acid, acetovanillone and mixtures thereof. These organic based mediators are preferably suited to be used with a laccase.

Transition metal coordination complexes can also be mediators. These compounds do not form radicals when oxidised by an oxidoreductase enzyme, and the electron exchange is centred on the metallic atom of the complex. This type of electron exchange involving only transition metal redox reactions allows the use of mediators with high stability in both oxidation states. This is a great advantage over the other type of mediators.

Several classes of peroxidase or oxidase mediators have been described, see US 5,700,769; and 5,965,510. Particular interest has been directed to the mediator phenothiazine-10-propionate. However, the described classes of mediators only enhance the peroxidase activity when hydrogen peroxide is added to the wash liquor. Other mediators are capable of enhancing the bleaching activity of the peroxidase enzyme with the addition of molecular oxygen, i.e. hydrogen peroxide does not need to be present for obtaining the desired enhancement of the oxidizing activity of peroxidases. Several classes of compounds can be envisaged which deliver the capability of enhancing the peroxidase activity, in the presence of only oxygen. Non-limiting examples include: the enhancer having the formula:

Z1HN-NHZ2

wherein Z1, is any organic group e. g. (substituted) - (hetero) (polycyclic)-aromatic, substituted (cyclo)-alkyl containing hetero atoms, and Z2 is electron withdrawing group, selected from the group consisting of optionally substituted alkyl/(hetero)aryl- -sulfone, sulfoxide, - sulfonate, - carbonyl, -oxalyl, - amidoxalyl, 5 hydrazidoxalyl, -carboxyl and esters and salts thereof, amidyl, - hydrazidyl, nitrile.

A suitable mediator may have the formula:

ArHN-NHZ2

wherein Z2 is as defined before and Ar is an optionally substituted aromatic or heteroaromatic group e.g. phenyl, phenyl substituted with halogen(s), alkoxy, alkyl, (alkyl)amino substituents, pyridinyl, alkyl- pyridinyl, furanyl. In one aspect, enhancer compounds may have the generic structures: 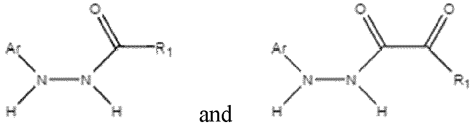 wherein the Ar group is as defined before and Rl is an optionally substituted alkyl, oxyalkyl, aryl, arylhydrazide, arylhydrazine or oxyaryl group, of interest are derivatives of 2'-phenylbenzohydrazide, having the following structure: 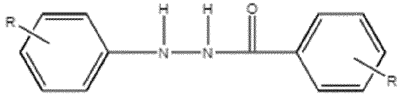 2-phenylhydrazide oxalate, having the following structure: 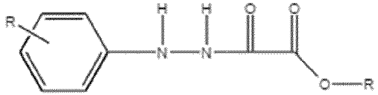 and oxalic acid bis(2-phenylhydrazide), having the following structure: 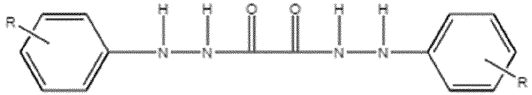 with R representing one or more substitutions independently selected from hydrogen, halogen(s), alkoxy, alkyl, (alkyl) amino, carbonate, carbonate ester, sulphonate, sulphonamide. Examples of such enhancers are: 2'-phenylbenzohydrazide; 2'-m-tolylbenzohydrazide; 2'-p-tolylbenzohydrazide; 2'-o-tolylbenzohydrazide; Ethyl [2-(m-tolyl)]hydrazide oxalate; Ethyl [2-(p-tolyl)]hydrazide oxalate; Ethyl [2-(o-tolyl)]hydrazide oxalate; Oxalic acid bis(2-phenylhydrazide); Oxalic acid bis(2-m-tolylhydrazide); and Oxalic acid bis(2-o- tolylhydrazide).

An especially preferred mediator for use herein is selected from the group consisting of phenoxazine-10-propionic acid, phenoxazine-10-hydroxy ethyl, phenothiazine-10-ethyl-4-carboxy, phenothiazine-10-propionic acid, promazine hydrochloride, phenothiazine-10-ethylalcohol and a mixture thereof.

### Cleaning composition

The cleaning composition of the method of the present invention, sometimes herein referred to as "the composition of the invention" is suitable for the cleaning of any type of surfaces when the cleaning involves the immersion of the surface in a wash liquor. The cleaning composition is suitable for use in hard surfaces and soft surfaces. It is particularly useful for use in laundry.

The cleaning composition of the invention would comprise the customary ingredients for the cleaning process, such as surfactants and builders. The cleaning composition would preferably comprise components which can be combined under the term cleaning aids and which comprise different active ingredient groups such as foam regulators, bleaches, bleach activators and enzymes. The composition, especially when the composition is for use in laundry, can comprise cleaning auxiliaries including substances which are intended to prevent dyed textiles from causing a change in colour impression after the wash (dye transfer inhibitors). This colour change of washed, i.e. clean, textiles can be due to the fact that dye components are removed from the fabric ("fading") by the washing process, and on the other hand, dyestuffs released from differently coloured fabrics can be deposited on the textile ("discolouring"). Other cleaning auxiliaries include electrolytes, pH regulators and in the case of compositions for use in laundry, optical brightener, dye transfer inhibitors, fragrances, etc.

The composition preferably contains a surfactant or a plurality of surfactants, particularly anionic surfactants, nonionic surfactants and mixtures thereof, but it can also comprise cationic, zwitterionic and amphoteric surfactants.

Preferably the composition of the invention is a laundry cleaning composition. A laundry cleaning composition is any composition suitable to be used in a fabric laundering operation. The laundry cleaning composition may be in the form of a powder, a liquid or a mixture thereof.

The cleaning composition may comprise between 10% and 60%, preferably between 15% and 55%, more preferably between 20% and 50%, most preferably between 25% and 45% by weight of the composition of a surfactant system. Preferably, the surfactant system comprises a non-soap surfactant. Preferably, the surfactant system comprises an anionic surfactant and optionally a non-ionic surfactant. More preferably, the weight ratio of anionic surfactant to non-ionic surfactant is from 1:2 to 20:1, preferably from 1:1 to 15: 1, more preferably from 1.5:1 to 10:1, most preferably from 5:1 to 10:1

The non-soap anionic surfactant is preferably selected from sulphate or sulphonate anionic surfactants or mixtures thereof, preferably linear alkylbenzene sulphonate, alkyl sulphate, alkoxylated alkyl sulphate or a mixture thereof. Preferably, the alkoxylated alkyl sulphate is an ethoxylated alkyl sulphate preferably with an average degree of ethoxylation of between 0.5 and 4, preferably between 1 and 4, more preferably between 2 and 4, most preferably about 3.

Preferably, the weight ratio of linear alkylbenzene sulphonate to alkoxylated alkyl sulphate is between 15:1 and 1:3, preferably 10:1 and 1:2, more preferably 5:1 and 1:1, even more preferably 3:1 and 1:1, most preferably 2:1 and 1:1.

The non-ionic surfactant may be selected from a fatty alcohol alkoxylate, an oxosynthesised fatty alcohol alkoxylate, Guerbet alcohol alkoxylates, alkyl phenol alcohol alkoxylates, alkyl polyglucoside or a mixture thereof. Preferably, the non-ionic surfactant comprises a fatty alcohol ethoxylate non-ionic surfactant. Even more preferably the nonionic surfactant consists of a fatty alcohol ethoxylate surfactant.

Suitable fatty alcohol ethoxylate nonionic surfactants include the condensation products of aliphatic alcohols with from 1 to 25 moles of ethylene oxide. The alkyl chain of the aliphatic alcohol can either be straight or branched, guerbet, primary or secondary, and generally contains from 8 to 22 carbon atoms. The starting alcohol can be naturally derived, e.g. starting from natural oils, or synthetically derived, e.g. alcohols obtained from for example oxo-, modified oxo- or Fischer-Tropsch processes. Examples of oxo-process derived fatty alcohols include the Lial and Isalchem 5 fatty alcohols ex Sasol company and Lutensol fatty alcohols ex BASF company.

Examples of modified-oxo process derived fatty alcohols include the Neodol fatty alcohols ex Shell company. Fischer-Tropsch derived fatty alcohols include Safol fatty alcohols ex Sasol company. The alkoxylate chain of fatty alcohol ethoxylates is made up solely of ethoxylate groups. Preferably, the fatty alcohol ethoxylate non-ionic surfactant comprises on average 10 between 8 and 18, more preferably between 10 and 16 even more preferably between 12 and 15 carbon atoms in the alcohol carbon chain, and on average between 5 and 12, preferably between 6 and 10, more preferably between 7 and 8 ethoxy units in the ethoxylation chain. Preferably, the weight ratio of linear alkylbenzene sulphonate to non-ionic surfactant is between 2:1 to 20:1 preferably 2:1 and 10:1; more preferably 5:1 and 10:1.

Preferably, the weight ratio of alkoxylated alkyl sulphate to non-ionic surfactant is between 2:1 and 20:1 preferably between 2:1 and 10:1 more preferably between 2:1 and 5:1. Preferably, the weight ratio of linear alkylbenzene sulphonate to fatty alcohol ethoxylate non-ionic surfactant is between 2:1 to 20:1 preferably 2:1 and 10:1; more preferably 5:1 and 10:1. Preferably, the weight ratio of alkoxylated alkyl sulphate to fatty alcohol ethoxylate nonionic surfactant is between 2:1 and 20:1 preferably between 2:1 and 10:1 more preferably between 2:1 and 5:1.

The cleaning composition may comprise polymers, preferably selected from alkoxylated, preferably ethoxylated polyethyleneimine, alkoxylated polyalkyl phenol, a polyester terephthalate, hydroxyethylcellulose, preferably quaternized hydroxyethylcellulose, a carboxymethylcellulose or a mixture thereof.

The cleaning composition may comprise an adjunct material, wherein the adjunct material is preferably selected from cleaning polymers, soil suspension polymers, surface modifying polymers, builders, chelants, dispersants, enzymes, enzyme stabilizers, catalytic materials, bleach, bleach activators, polymeric dispersing agents, anti-redeposition agents, suds suppressors, aesthetic dyes, opacifiers, perfumes, perfume delivery systems, structurants, hydrotropes, rheology modifiers, processing aids, pigments and mixtures thereof. Having an adjunct material in the composition provides good overall cleaning, soil suspension and whiteness or colour brightness profile of the fabric to be treated.

Method of Use. In the method of the present invention, the surface to be cleaned is typically contacted with the wash liquor in a domestic or industrial washing process. Examples include hand-washing or in an automatic washing machine or process. The wash liquor comprising cleaning composition and supporting substrate having oxidoreductase enzyme immobilized thereon is contacted, preferably under agitation, with the surfaces to be cleaned. An effective amount of the cleaning composition herein added to water to form the wash liquor aqueous may comprise amounts sufficient to form from about 500 to 25,000 ppm, or from 500 to 15,000 ppm of cleaning composition in aqueous washing solution, or from about 1,000 to 3,000 ppm of the detergent compositions herein will be provided in aqueous washing solution, or wherein the concentration of the cleaning composition in the wash liquor is from above 0.001g/l to 5g/l, or from 1g/l, and to 4.5g/l, or to 4.0g/l, or to 3.5g/l, or to 3.0g/l, or to 2.5g/l, or even to 2.0g/l, or even to 1.5g/l.

In one aspect, such method comprises the steps of optionally washing and/or rinsing said surface, contacting said surface with the cleaning composition and immobilized enzyme disclosed in this specification then optionally washing and/or rinsing said surface, with an optional drying step. In a preferred method, following the wash step in which the surface is cleaned by contacting the surface with the wash liquor, the supporting substrate and oxidoreductase enzyme are separated from the wash liquor and re-used in a second wash step for cleaning a second surface.

Fabric surfaces suitable for the present invention comprise natural or synthetic textiles such as cotton, wool, silk, polyester and nylon and especially for treatment of mixed fabrics and/or fibres comprising synthetic and cellulosic fabrics and/or fibres. As examples of synthetic fabrics are polyester, nylon, these may be present in mixtures with cellulosic fibres, for example, poly cotton fabrics. The solution typically has a pH of from 7 to 11, more usually 8 to 10.5. The water temperatures typically range from about 5 °C to about 90 °C. The water to fabric ratio is typically from about 1:1 to about 30:1.

### EXAMPLES

### Example 1

Immobilization of an oxidoreductase enzyme onto a solid substrate is done using EDC (N-(3-Dimethylaminopropyl)-N'ethylcarbodiimide) as described by Fischer MJ (Methods Mol Biol. 2010, 627:55-73). The activity of the immobilized oxidoreductase enzyme sample is confirmed by adding 600µl of syringaldazine onto 250ml of a liquid detergent solution. 0.06ppm of immobilized oxidoreductase enzyme is added to the solution and absorbance at 531nm is measured over a certain period of time. The starting colour of the solution is colourless/yellow with the end point being purple.

| Time (min) | Abs (nm) |
|---|---|
| 0 | 0.058 |
| 0.5 | 0.063 |
| 1 | 0.068 |
| 2 | 0.073 |
| 3 | 0.081 |
| 4 | 0.087 |
| 5 | 0.095 |
| 10 | 0.126 |
| 15 | 0.15 |
| 20 | 0.164 |
| 25 | 0.167 |

Cotton and poly cotton fabrics including white and mixed coloured fabrics are washed together in a wash step comprising detergent composition 1. The wash water contains 13 litres water and from 30 to 60 g of detergent 1. The wash liquor also contains a sample of the immobilised enzyme described above.

The following are illustrative examples of cleaning compositions of the invention and are not intended to be limiting. The invention is as defined by the claims.

### Detergent Composition Examples 1-7: Heavy Duty Liquid laundry detergent compositions.

| **Ingredients** | **1** | **2** | **3** | **4** | **5** | **6** | **7** |
|---|---|---|---|---|---|---|---|
| | % weight | | | | | | |
| AE_{1.8}S | 6.77 | 5.16 | 1.36 | 1.30 | - | - | - |
| AE₃S | - | - | - | - | 0.45 | - | - |
| LAS | 0.86 | 2.06 | 2.72 | 0.68 | 0.95 | 1.56 | 3.55 |
| HSAS | 1.85 | 2.63 | 1.02 | - | - | - | - |
| AE9 | 6.32 | 9.85 | 10.20 | 7.92 | | | |
| AE8 | | | | | | | 35.45 |
| AE7 | | | | | 8.40 | 12.44 | |
| C₁₂₋₁₄ dimethyl Amine Oxide | 0.30 | 0.73 | 0.23 | 0.37 | - | - | - |
| C₁₂₋₁₈ Fatty Acid | 0.80 | 1.90 | 0.60 | 0.99 | 1.20 | - | 15.00 |
| Citric Acid | 2.50 | 3.96 | 1.88 | 1.98 | 0.90 | 2.50 | 0.60 |
| Optical Brightener 1 | 1.00 | 0.80 | 0.10 | 0.30 | 0.05 | 0.50 | 0.001 |
| Optical Brightener 3 | 0.001 | 0.05 | 0.01 | 0.20 | 0.50 | - | 1.00 |
| Sodium formate | 1.60 | 0.09 | 1.20 | 0.04 | 1.60 | 1.20 | 0.20 |
| DTI 1 | 0.32 | 0.05 | - | 0.60 | 0.10 | 0.60 | 0.01 |
| DTI 2 | 0.32 | 0.10 | 0.60 | 0.60 | 0.05 | 0.40 | 0.20 |
| Sodium hydroxide | 2.30 | 3.80 | 1.70 | 1.90 | 1.70 | 2.50 | 2.30 |
| Monoethanolamine | 1.40 | 1.49 | 1.00 | 0.70 | - | - | - |
| Diethylene glycol | 5.50 | - | 4.10 | - | - | - | - |
| Chelant 1 | 0.15 | 0.15 | 0.11 | 0.07 | 0.50 | 0.11 | 0.80 |
| 4-formyl-phenylboronic acid | - | - | - | - | 0.05 | 0.02 | 0.01 |
| Sodium tetraborate | 1.43 | 1.50 | 1.10 | 0.75 | - | 1.07 | - |
| Ethanol | 1.54 | 1.77 | 1.15 | 0.89 | - | 3.00 | 7.00 |
| Polymer 1 | 0.10 | - | - | - | - | - | 2.00 |
| Polymer 2 | 0.30 | 0.33 | 0.23 | 0.17 | - | - | - |
| Polymer 3 | - | - | - | - | - | - | 0.80 |
| Polymer 4 | 0.80 | 0.81 | 0.60 | 0.40 | 1.00 | 1.00 | - |
| 1,2-Propanediol | - | 6.60 | - | 3.30 | 0.50 | 2.00 | 8.00 |
| Structurant | 0.10 | - | - | - | - | - | 0.10 |
| Perfume | 1.60 | 1.10 | 1.00 | 0.80 | 0.90 | 1.50 | 1.60 |
| Perfume encapsulate | 0.10 | 0.05 | 0.01 | 0.02 | 0.10 | 0.05 | 0.10 |
| Protease | 0.80 | 0.60 | 0.70 | 0.90 | 0.70 | 0.60 | 1.50 |
| Mannanase | 0.07 | 0.05 | 0.045 | 0.06 | 0.04 | 0.045 | 0.10 |
| Amylase 1 | 0.30 | - | 0.30 | 0.10 | - | 0.40 | 0.10 |
| Amylase 2 | - | 0.20 | 0.10 | 0.15 | 0.07 | - | 0.10 |
| Xyloglucannase | 0.20 | 0.10 | - | - | 0.05 | 0.05 | 0.20 |
| Lipase | 0.40 | 0.20 | 0.30 | 0.10 | 0.20 | - | - |
| Polishing enzyme | - | 0.04 | - | - | - | 0.004 | - |
| Nuclease | 0.05 | 0.03 | 0.01 | 0.03 | 0.03 | 0.003 | 0.003 |
| Dispersin B | - | - | - | 0.05 | 0.03 | 0.001 | 0.001 |
| Acid Violet 50 | 0.05 | - | - | - | - | - | 0.005 |
| Direct Violet 9 | - | - | - | - | - | 0.05 | - |
| Violet DD | - | 0.035 | 0.02 | 0.037 | 0.04 | - | - |
| Immobilized oxidoreductase enzyme | 0.5 | 0.03 | 0.005 | 0.05 | 0.5 | 0.03 | 0.005 |
| Oxidoreductase-mediator | 0.05 | - | - | - | 0.05 | - | - |
| Water, dyes & minors | Balance | | | | | | |
| pH | 8.2 | | | | | | |

Based on total cleaning and/or treatment composition weight. Enzyme levels are reported as raw material.

### Detergent Composition Examples 8 to 18: Unit Dose Compositions.

These examples provide various formulations for unit dose laundry detergents. Compositions 8 to 12 comprise a single unit dose compartment. The film used to encapsulate the compositions is a polyvinyl-alcohol-based film.

| **Ingredients** | **8** | **9** | **10** | **11** | **12** |
|---|---|---|---|---|---|
| | **% weight** | | | | |
| LAS | 19.09 | 16.76 | 8.59 | 6.56 | 3.44 |
| AE3S | 1.91 | 0.74 | 0.18 | 0.46 | 0.07 |
| AE7 | 14.00 | 17.50 | 26.33 | 28.08 | 31.59 |
| Citric Acid | 0.6 | 0.6 | 0.6 | 0.6 | 0.6 |
| C12-15 Fatty Acid | 14.8 | 14.8 | 14.8 | 14.8 | 14.8 |
| Polymer 3 | 4.0 | 4.0 | 4.0 | 4.0 | 4.0 |
| Chelant 2 | 1.2 | 1.2 | 1.2 | 1.2 | 1.2 |
| Optical Brightener 1 | 0.20 | 0.25 | 0.01 | 0.01 | 0.50 |
| Optical Brightener 2 | 0.20 | - | 0.25 | 0.03 | 0.01 |
| Optical Brightener 3 | 0.18 | 0.09 | 0.30 | 0.01 | - |
| DTI 1 | 0.10 | - | 0.20 | 0.01 | 0.05 |
| DTI 2 | - | 0.10 | 0.20 | 0.25 | 0.05 |
| Glycerol | 6.1 | 6.1 | 6.1 | 6.1 | 6.1 |
| Monoethanol amine | 8.0 | 8.0 | 8.0 | 8.0 | 8.0 |
| Tri-isopropanol amine | - | - | 2.0 | - | - |
| Tri-ethanol amine | - | 2.0 | - | - | - |
| Cumene sulfonate | - | - | - | - | 2.0 |
| Protease | 0.80 | 0.60 | 0.07 | 1.00 | 1.50 |
| Mannanase | 0.07 | 0.05 | 0.05 | 0.10 | 0.01 |
| Amylase 1 | 0.20 | 0.11 | 0.30 | 0.50 | 0.05 |
| Amylase 2 | 0.11 | 0.20 | 0.10 | - | 0.50 |
| Polishing enzyme | 0.005 | 0.05 | - | - | - |
| Nuclease | 0.005 | 0.05 | 0.005 | 0.010 | 0.005 |
| Dispersin B | 0.010 | 0.05 | 0.005 | 0.005 | - |
| Cyclohexyl dimethanol | - | - | - | 2.0 | - |
| Acid violet 50 | 0.03 | 0.02 | | | |
| Violet DD | | | 0.01 | 0.05 | 0.02 |
| Structurant | 0.14 | 0.14 | 0.14 | 0.14 | 0.14 |
| Perfume | 1.9 | 1.9 | 1.9 | 1.9 | 1.9 |
| Immobilized oxidoreductase enzyme | 0.5 | 0.03 | 0.005 | 0.05 | 0.5 |
| Oxidoreductase-mediator | 0.05 | - | - | - | 0.05 |
| Water and miscellaneous | To 100% | | | | |
| pH | 7.5-8.2 | | | | |

Based on total cleaning and/or treatment composition weight. Enzyme levels are reported as raw material.

In the following examples the unit dose has three compartments, but similar compositions can be made with two, four or five compartments. The film used to encapsulate the compartments is polyvinyl alcohol.

Based on total cleaning and/or treatment composition weig

| **Base compositions Ingredients** | **13** | **14** | **15** | **16** |
|---|---|---|---|---|
| | **% weight** | | | |
| HLAS | 26.82 | 16.35 | 7.50 | 3.34 |
| AE7 | 17.88 | 16.35 | 22.50 | 30.06 |
| Citric Acid | 0.5 | 0.7 | 0.6 | 0.5 |
| C12-15 Fatty acid | 16.4 | 6.0 | 11.0 | 13.0 |
| Polymer 1 | 2.9 | 0.1 | - | - |
| Polymer 3 | 1.1 | 5.1 | 2.5 | 4.2 |
| Cationic cellulose polymer | - | - | 0.3 | 0.5 |
| Polymer 6 | - | 1.5 | 0.3 | 0.2 |
| Chelant 2 | 1.1 | 2.0 | 0.6 | 1.5 |
| Optical Brightener 1 | 0.20 | 0.25 | 0.01 | 0.005 |
| Optical Brightener 3 | 0.18 | 0.09 | 0.30 | 0.005 |
| DTI 1 | 0.1 | - | 0.2 | - |
| DTI 2 | - | 0.1 | 0.2 | - |
| Glycerol | 5.3 | 5.0 | 5.0 | 4.2 |
| Monoethanolamine | 10.0 | 8.1 | 8.4 | 7.6 |
| Polyethylene glycol | - | - | 2.5 | 3.0 |
| Potassium sulfite | 0.2 | 0.3 | 0.5 | 0.7 |
| Protease | 0.80 | 0.60 | 0.40 | 0.80 |
| Amylase 1 | 0.20 | 0.20 | 0.200 | 0.30 |
| Polishing enzyme | - | - | 0.005 | 0.005 |
| Nuclease | 0.05 | 0.010 | 0.005 | 0.005 |
| Dispersin B | - | 0.010 | 0.010 | 0.010 |
| MgCl₂ | 0.2 | 0.2 | 0.1 | 0.3 |
| Structurant | 0.2 | 0.1 | 0.2 | 0.2 |
| Acid Violet 50 | 0.04 | 0.03 | 0.05 | 0.03 |
| Perfume / encapsulates | 0.10 | 0.30 | 0.01 | 0.05 |
| Immobilized oxidoreductase enzyme | 0.5 | 0.03 | 0.05 | 0.5 |
| Oxidoreductase-mediator | 0.05 | - | - | 0.05 |
| Solvents and misc. | To 100% | | | |
| pH | 7.0-8.2 | | | |

| **Finishing compositions** | **17** | | | **18** | | |
|---|---|---|---|---|---|---|
| Compartment | A | B | C | A | B | C |
| Volume of each compartment | 40 ml | 5 ml | 5 ml | 40 ml | 5 ml | 5 ml |
| **Ingredients** | Active material in Wt.% | | | | | |
| Perfume | 1.6 | 1.6 | 1.6 | 1.6 | 1.6 | 1.6 |
| Violet DD | 0 | 0.006 | 0 | 0 | 0.004 | - |
| TiO2 | - | - | 0.1 | - | | 0.1 |
| Sodium Sulfite | 0.4 | 0.4 | 0.4 | 0.3 | 0.3 | 0.3 |
| Polymer 5 | - | | | 2 | - | - |
| Hydrogenated castor oil | 0.14 | 0.14 | 0.14 | 0.14 | 0.14 | 0.14 |
| Base Composition 13, 14, 15 or 16 | Add to 100% | | | | | |

ht, enzyme levels are reported as raw material.

Detergent Composition Examples 19 to 24:Granular laundry detergent compositions for hand washing or washing machines, typically top-loading washing machines.

| **Ingredient** | **19** | **20** | **21** | **22** | **23** | **24** |
|---|---|---|---|---|---|---|
| | **% weight** | | | | | |
| LAS | 11.33 | 10.81 | 7.04 | 4.20 | 3.92 | 2.29 |
| Quaternary ammonium | 0.70 | 0.20 | 1.00 | 0.60 | - | - |
| AE3S | 0.51 | 0.49 | 0.32 | - | 0.08 | 0.10 |
| AE7 | 8.36 | 11.50 | 12.54 | 11.20 | 16.00 | 21.51 |
| Sodium Tripolyphosphate | 5.0 | - | 4.0 | 9.0 | 2.0 | - |
| Zeolite A | - | 1.0 | - | 1.0 | 4.0 | 1.0 |
| Sodium silicate 1.6R | 7.0 | 5.0 | 2.0 | 3.0 | 3.0 | 5.0 |
| Sodium carbonate | 20.0 | 17.0 | 23.0 | 14.0 | 14.0 | 16.0 |
| Polyacrylate MW 4500 | 1.0 | 0.6 | 1.0 | 1.0 | 1.5 | 1.0 |
| Polymer 6 | 0.1 | 0.2 | - | - | 0.1 | - |
| Carboxymethyl cellulose | 1.0 | 0.3 | 1.0 | 1.0 | 1.0 | 1.0 |
| Acid Violet 50 | 0.05 | - | 0.02 | - | 0.04 | - |
| Violet DD | - | 0.03 | - | 0.03 | - | 0.03 |
| Protease 2 | 0.10 | 0.10 | 0.10 | 0.10 | - | 0.10 |
| Amylase | 0.03 | - | 0.03 | 0.03 | 0.03 | 0.03 |
| Lipase | 0.03 | 0.07 | 0.30 | 0.10 | 0.07 | 0.40 |
| Polishing enzyme | 0.002 | - | 0.05 | - | 0.02 | - |
| Nuclease | 0.001 | 0.001 | 0.01 | 0.05 | 0.002 | 0.02 |
| Dispersin B | 0.001 | 0.001 | 0.05 | - | 0.001 | - |
| Optical Brightener 1 | 0.200 | 0.001 | 0.300 | 0.650 | 0.050 | 0.001 |
| Optical Brightener 2 | 0.060 | - | 0.650 | 0.180 | 0.200 | 0.060 |
| Optical Brightener 3 | 0.100 | 0.060 | 0.050 | - | 0.030 | 0.300 |
| Chelant 1 | 0.60 | 0.80 | 0.60 | 0.25 | 0.60 | 0.60 |
| DTI 1 | 0.32 | 0.15 | 0.15 | - | 0.10 | 0.10 |
| DTI 2 | 0.32 | 0.15 | 0.30 | 0.30 | 0.10 | 0.20 |
| Sodium Percarbonate | - | 5.2 | 0.1 | - | - | - |
| Sodium Perborate | 4.4 | - | 3.85 | 2.09 | 0.78 | 3.63 |
| Nonanoyloxybenzensulfonate | 1.9 | 0.0 | 1.66 | 0.0 | 0.33 | 0.75 |
| Tetraacetylehtylenediamine | 0.58 | 1.2 | 0.51 | 0.0 | 0.015 | 0.28 |
| Photobleach | 0.0030 | 0.0 | 0.0012 | 0.0030 | 0.0021 | - |
| S-ACMC | 0.1 | 0.0 | 0.0 | 0.0 | 0.06 | 0.0 |
| Immobilized oxidoreductase enzyme | 0.5 | 0.05 | 0.1 | 0.05 | 0.5 | 0.05 |
| Oxidoreductase-mediator | 0.05 | - | - | - | 0.05 | - |
| Sulfate/Moisture | Balance | | | | | |

Detergent Composition Examples 25-30: Granular laundry detergent compositions typically for front-loading automatic washing machines.

| **Ingredient** | **25** | **26** | **27** | **28** | **29** | **30** |
|---|---|---|---|---|---|---|
| | **% weight** | | | | | |
| LAS | 6.08 | 5.05 | 4.27 | 3.24 | 2.30 | 1.09 |
| AE3S | - | 0.90 | 0.21 | 0.18 | - | 0.06 |
| AS | 0.34 | - | - | - | - | - |
| AE7 | 4.28 | 5.95 | 6.72 | 7.98 | 9.20 | 10.35 |
| Quaternary ammonium | 0.5 | - | - | 0.3 | - | - |
| Crystalline layered silicate | 4.1 | - | 4.8 | - | - | - |
| Zeolite A | 5.0 | - | 2.0 | - | 2.0 | 2.0 |
| Citric acid | 3.0 | 4.0 | 3.0 | 4.0 | 2.5 | 3.0 |
| Sodium carbonate | 11.0 | 17.0 | 12.0 | 15.0 | 18.0 | 18.0 |
| Sodium silicate 2R | 0.08 | - | 0.11 | - | - | - |
| Optical Brightener 1 | - | 0.25 | 0.05 | 0.01 | 0.10 | 0.02 |
| Optical Brightener 2 | - | - | 0.25 | 0.20 | 0.01 | 0.08 |
| Optical Brightener 3 | - | 0.06 | 0.04 | 0.15 | - | 0.05 |
| DTI 1 | 0.08 | - | 0.04 | - | 0.10 | 0.01 |
| DTI 2 | 0.08 | - | 0.04 | 0.10 | 0.10 | 0.02 |
| Soil release agent | 0.75 | 0.72 | 0.71 | 0.72 | - | - |
| Acrylic /maleic acid copolymer | 1.1 | 3.7 | 1.0 | 3.7 | 2.6 | 3.8 |
| Carboxymethyl cellulose | 0.2 | 1.4 | 0.2 | 1.4 | 1.0 | 0.5 |
| Protease 3 | 0.20 | 0.20 | 0.30 | 0.15 | 0.12 | 0.13 |
| Amylase 3 | 0.20 | 0.15 | 0.20 | 0.30 | 0.15 | 0.15 |
| Lipase | 0.05 | 0.15 | 0.10 | - | - | - |
| Amylase 2 | 0.03 | 0.07 | - | - | 0.05 | 0.05 |
| Cellulase 2 | - | - | - | - | 0.10 | 0.10 |
| Polishing enzyme | 0.003 | 0.005 | 0.020 | - | - | - |
| Nuclease | 0.002 | 0.010 | 0.020 | 0.020 | 0.010 | 0.003 |
| Dispersin B | 0.002 | 0.010 | 0.020 | 0.020 | 0.010 | 0.002 |
| Tetraacetylehtylenediamine | 3.6 | 4.0 | 3.6 | 4.0 | 2.2 | 1.4 |
| Sodium percabonate | 13.0 | 13.2 | 13.0 | 13.2 | 16.0 | 14.0 |
| Chelant 3 | - | 0.2 | - | 0.2 | - | 0.2 |
| Chelant 2 | 0.2 | - | 0.2 | - | 0.2 | 0.2 |
| MgSO₄ | - | 0.42 | - | 0.42 | - | 0.4 |
| Perfume | 0.5 | 0.6 | 0.5 | 0.6 | 0.6 | 0.6 |
| Suds suppressor agglomerate | 0.05 | 0.10 | 0.05 | 0.10 | 0.06 | 0.05 |
| Soap | 0.45 | 0.45 | 0.45 | 0.45 | - | - |
| Acid Violet 50 | 0.04 | - | 0.05 | - | 0.04 | - |
| Violet DD | - | 0.04 | - | 0.05 | - | 0.04 |
| S-ACMC | 0.01 | 0.01 | - | 0.01 | - | - |
| Direct Violet 9 (active) | - | - | 0.0001 | 0.0001 | - | - |
| Immobilized oxidoreductase enzyme | 0.5 | 0.1 | 0.05 | 0.05 | 0.5 | 0.1 |
| Oxidoreductase-mediator | 0.2 | - | - | - | 0.2 | - |
| Sulfate/ Water & Miscellaneous | Balance | | | | | |

| | |
|---|---|
| AE1.8S | is C₁₂₋₁₅ alkyl ethoxy (1.8) sulfate |
| AE3S | is C₁₂₋₁₅ alkyl ethoxy (3) sulfate |
| AE7 | is C₁₂₋₁₃ alcohol ethoxylate, with an average degree of ethoxylation of 7 |
| AE8 | is C₁₂₋₁₃ alcohol ethoxylate, with an average degree of ethoxylation of 8 |
| AE9 | is C₁₂₋₁₃ alcohol ethoxylate, with an average degree of ethoxylation of 9 |
| Amylase 1 | is Stainzyme^{®}, 15 mg active/g |
| Amylase 2 | is Natalase^{®}, 29 mg active/g |
| Amylase 3 | is Stainzyme Plus^{®}, 20 mg active/g, |
| AS | is C₁₂₋₁₄ alkylsulfate |
| Cellulase 2 | is Celluclean^{™} , 15.6 mg active/g |
| Xyloglucanase | is Whitezyme^{®}, 20mg active/g |
| Chelant 1 | is diethylene triamine pentaacetic acid |
| Chelant 2 | is 1-hydroxyethane 1,1-diphosphonic acid |
| Chelant 3 | is sodium salt of ethylenediamine-N,N'-disuccinic acid, (S,S) isomer (EDDS) |
| Dispersin B | is a glycoside hydrolase, reported as 1000mg active/g |
| DTI 1 | is poly(4-vinylpyridine-1-oxide) (such as Chromabond S-403E^{®}), |
| DTI 2 | is poly(1-vinylpyrrolidone-co-1-vinylimidazole) (such as Sokalan HP56^{®} ). |
| Dye control agent | Dye control agent in accordance with the invention, for example Suparex^{®} O.IN (M1), Nylofixan^{®} P (M2), Nylofixan^{®} PM (M3), or Nylofixan^{®} HF (M4) |
| HSAS | is mid-branched alkyl sulfate as disclosed in US 6,020,303 and US6,060,443 |

### Immobilized oxidoreductase

| | |
|---|---|
| enzyme | is immobilized oxidoreductase enzyme in accordance with the invention; for example, Guardzyme^{®} 10.5mg/g. |
| LAS | is linear alkylbenzenesulfonate having an average aliphatic carbon chain length C₉-C₁₅ (HLAS is acid form). |
| Lipase | is Lipex^{®}, 18 mg active/g |
| Mannanase | is Mannaway^{®}, 25 mg active/g |
| Nuclease | is a Phosphodiesterase SEQ ID NO 1, reported as 1000mg active/g |
| Optical Brightener 1 | is disodium 4,4'-bis{[4-anilino-6-morpholino-s-triazin-2-yl]-amino}-2,2'-stilbenedisulfonate |
| Optical Brightener 2 | is disodium 4,4'-bis-(2-sulfostyryl)biphenyl (sodium salt) |
| Optical Brightener 3 | is Optiblanc SPL10^{®} from 3V Sigma |
| Oxidoreductase-mediator | is methyl syringate |
| Perfume encapsulate | is a core-shell melamine formaldehyde perfume microcapsules. |
| Photobleach | is a sulfonated zinc phthalocyanine |
| Polishing enzyme | is Para-nitrobenzyl esterase, reported as 1000mg active/g |
| Polymer 1 | is bis((C₂H₅O)(C₂H₄O)n)(CH₃)-N⁺-CₓH₂ₓ-N⁺-(CH₃)-bis((C₂H₅O)(C₂H₄O)n), wherein n = 20-30,x = 3 to 8 or sulphated or sulfonated variants thereof |
| Polymer 2 | is ethoxylated (EO₁₅) tetraethylene pentamine |
| Polymer 3 | is ethoxylated polyethylenimine |
| Polymer 4 | is ethoxylated hexamethylene diamine |
| Polymer 5 | is Acusol 305, provided by Rohm&Haas |
| Polymer 6 | is a polyethylene glycol polymer grafted with vinyl acetate side chains, provided by BASF. |
| Protease | is Purafect Prime^{®}, 40.6 mg active/g |
| Protease 2 | is Savinase^{®}, 32.89 mg active/g |
| Protease 3 | is Purafect^{®}, 84 mg active/g |
| Quaternary ammonium | is C₁₂₋₁₄ Dimethylhydroxyethyl ammonium chloride |
| S-ACMC | is Reactive Blue 19 Azo-CM-Cellulose provided by Megazyme |
| Soil release agent | is Repel-o-tex^{®} SF2 |
| Structurant | is Hydrogenated Castor Oil |
| Violet DD | is a thiophene azo dye provided by Milliken |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method for cleaning a surface comprising contacting the surface with a wash liquor, the wash liquor comprising a cleaning composition; a supporting substrate; and an oxidoreductase enzyme, **characterised in that** the oxidoreductase enzyme is immobilised on the supporting substrate by means of a chemical bond.

2. A method according to claim 1 wherein the oxidoreductase enzyme is selected from the group consisting of laccase, peroxidase and a mixture thereof.

3. A method according to claim 1 or claim 2 wherein the oxidoreductase enzyme is immobilised on the supporting substrate by means of covalent bond.

4. A method according to any preceding claim wherein the supporting substrate is selected from the group consisting of fabrics, non-woven materials and plastics.

5. A method according to any preceding claim wherein the supporting substrate is a tri-dimensional hollow body wherein the peroxidase is immobilized on the interior surface of the hollow body.

6. A method according to any preceding claim wherein the supporting substrate is an inorganic particle.

7. A method according to any preceding claim wherein the cleaning composition comprises from 0.01% to 15% by weight of the composition of a peroxygen source and the oxidoreductase enzyme is a peroxidase.

8. A method according to claim 7 wherein the peroxygen source is selected from the group consisting of hydrogen peroxide, a hydrogen peroxide precursor, a hydrogen peroxide generating enzyme system, or a peroxycarboxylic acid or a salt thereof and mixtures thereof.

9. A method according to any preceding claim wherein the wash liquor comprises a mediator, preferably selected from the group consisting of organic-based mediators, transition metal coordination complex mediators and mixtures thereof.

10. A method according to claim 9 wherein the mediator is immobilized on a supporting substrate.

11. A method according to claim 9 or claim 10 wherein the oxidoreductase enzyme is a peroxidase and the mediator is a peroxidase mediator having the formula
Z1HN-NHZ2
wherein Z1, is any organic group e. g. (substituted) - (hetero) (polycyclic)-aromatic, substituted (cyclo)-alkyl containing hetero atoms, and Z2 is electron withdrawing group, selected from the group consisting of optionally substituted alkyl/(hetero)aryl- -sulfone, sulfoxide, - sulfonate, -carbonyl, -oxalyl, - amidoxalyl, 5 hydrazidoxalyl, -carboxyl and esters and salts thereof, amidyl, -hydrazidyl, nitrile.

12. A method according to claim 9 or claim 10 wherein the oxidoreductase enzyme is a peroxidase and wherein the mediator is a peroxidase mediator selected from the group consisting of phenoxazine-10-propionic acid, phenoxazine-10-hydroxyethyl, phenothiazine-10-ethyl-4-carboxy, phenothiazine-10-propionic acid, promazine hydrochloride, phenothiazine-10-ethylalcohol and a mixture thereof.

13. A method according to any preceding claim wherein the oxidoreductase enzyme comprises a laccase enzyme, preferably the wash liquor additionally comprises a laccase mediator selected from the group consisting of 2,2'-azinobis-(3-ethylbenzthiazoline-6-sulfonate), 1-hydroxybenzotriazole, violuric acid, N-hydroxyacetanilide, methyl syringate, acetosyringone, syringaldezine, butyl syringate, pentyl syringate, hexyl syringate, heptyl syringate, vanillyl alcohol, synapic acid, acetovanillone, and mixtures thereof.

14. A method according to any preceding claim wherein the cleaning composition comprises a surfactant system comprising an anionic surfactant and optionally a non-ionic surfactant.

15. A method according to any preceding claim wherein the surface comprises fabric and the method is for laundering of fabrics and wherein the fabrics comprise mixed colour fabrics, and preferably following the wash step in which the surface is cleaned by contacting the surface with the wash liquor, the supporting substrate and oxidoreductase enzyme are separated from the wash liquor and re-used in a second wash step for cleaning a second surface.

## Patentansprüche

1. Verfahren zum Reinigen einer Oberfläche, umfassend ein Inkontaktbringen der Oberfläche mit einer Waschflotte, die Waschflotte umfassend eine Reinigungszusammensetzung; ein Trägersubstrat; und ein Oxidoreductase-Enzym, **dadurch gekennzeichnet, dass** das Oxidoreductase-Enzym mittels einer chemischen Bindung auf dem Trägersubstrat immobilisiert ist.

2. Verfahren nach Anspruch 1, wobei das Oxidoreductase-Enzym ausgewählt ist aus der Gruppe bestehend aus Laccase, Peroxidase und einer Mischung davon.

3. Verfahren nach Anspruch 1 oder 2, wobei das Oxidoreductase-Enzym mittels einer kovalenten Bindung auf dem Trägersubstrat immobilisiert ist.

4. Verfahren nach einem der vorstehenden Ansprüche, wobei das Trägersubstrat ausgewählt ist aus der Gruppe bestehend aus Stoffen, Vliesmaterialien und Kunststoffen.

5. Verfahren nach einem der vorstehenden Ansprüche, wobei das Trägersubstrat ein dreidimensionaler Hohlkörper ist, wobei die Peroxidase auf der Innenoberfläche des Hohlkörpers immobilisiert ist.

6. Verfahren nach einem der vorstehenden Ansprüche, wobei das Trägersubstrat ein anorganisches Teilchen ist.

7. Verfahren nach einem der vorstehenden Ansprüche, wobei die Reinigungszusammensetzung von zu 0,01 Gew.-% bis 15 Gew.-% der Zusammensetzung eine Peroxygen-Quelle umfasst und das Oxidoreductase-Enzym eine Peroxidase ist.

8. Verfahren nach Anspruch 7, wobei die Peroxygen-Quelle ausgewählt ist aus der Gruppe bestehend aus Wasserstoffperoxid, einem Wasserstoffperoxid-Vorläufer, einem wasserstoffperoxid-erzeugenden Enzymsystem oder einer Peroxycarbonsäure oder einem Salz davon und Mischungen davon.

9. Verfahren nach einem der vorstehenden Ansprüche, wobei die Waschflotte einen Mediator umfasst, vorzugsweise ausgewählt aus der Gruppe bestehend aus Mediatoren auf organischer Basis, Übergangsmetallkoordinationskomplexmediatoren und Mischungen davon.

10. Verfahren nach Anspruch 9, wobei der Mediator auf einem Trägersubstrat immobilisiert ist.

11. Verfahren nach Anspruch 9 oder 10, wobei das Oxidoreductase-Enzym eine Peroxidase ist und der Mediator ein Peroxidase-Mediator ist, der die Formel aufweist
Z1HN-NHZ2
wobei Z1 eine beliebige organische Gruppe ist, z. B. (substituiert)-(hetero)(polycyclisch)-aromatisch, substituiertes (Cyclo)alkyl, das Heteroatome enthält, und Z2 eine elektronenentziehende Gruppe ist, ausgewählt aus der Gruppe bestehend aus wahlweise substituiertem Alkyl/(Hetero)aryl-, -sulfon, Sulfoxid, -sulfonat, -carbonyl, -oxalyl, -amidoxalyl, 5-Hydrazidoxalyl, -carboxyl und Estern und Salzen davon, Amidyl, -hydrazidyl, Nitril.

12. Verfahren nach Anspruch 9 oder 10, wobei das Oxidoreductase-Enzym eine Peroxidase ist und wobei der Mediator ein Peroxidase-Mediator ist, ausgewählt aus der Gruppe bestehend aus Phenoxazin-10-propionsäure, Phenoxazin-10-hydroxyethyl, Phenothiazin-10-ethyl-4-carboxy, Phenothiazin-10-propionsäure, Promazinhydrochlorid, Phenothiazin-10-ethylalkohol und einer Mischung davon.

13. Verfahren nach einem der vorstehenden Ansprüche, wobei das Oxidoreductase-Enzym ein Laccase-Enzym umfasst, wobei vorzugsweise die Waschflotte zusätzlich einen Laccase-Mediator umfasst, ausgewählt aus der Gruppe bestehend aus 2,2'-Azinobis-(3-ethylbenzthiazolin-6-sulfonat), 1-Hydroxybenzotriazol, Violursäure, N-Hydroxyacetanilid, Methylsyringat, Acetosyringon, Syringaldezin, Butylsyringat, Pentylsyringat, Hexylsyringat, Heptylsyringat, Vanillylalkohol, Sinapinsäure, Acetovanillon und Mischungen davon.

14. Verfahren nach einem der vorstehenden Ansprüche, wobei die Reinigungszusammensetzung ein Tensid-System umfasst, umfassend ein anionisches Tensid und wahlweise ein nichtionisches Tensid.

15. Verfahren nach einem der vorstehenden Ansprüche, wobei die Oberfläche Stoff umfasst und das Verfahren für einen Waschvorgang von Stoffen dient und wobei die Stoffe Stoffe aus gemischten Farben umfassen und wobei vorzugsweise folgend auf den Waschschritt, in dem die Oberfläche durch Inkontaktbringen der Oberfläche mit der Waschflotte gereinigt wird, das Trägersubstrat und das Oxidoreductase-Enzym von der Waschflotte getrennt und in einem zweiten Waschschritt zum Reinigen einer zweiten Oberfläche wiederverwendet werden.

## Revendications

1. Procédé permettant de nettoyer une surface comprenant la mise en contact de la surface avec une lessive, la lessive comprenant une composition de nettoyage ; un substrat de support ; et une enzyme oxydo-réductase, **caractérisé en ce que** l'enzyme oxydo-réductase est immobilisée sur le substrat de support au moyen d'une liaison chimique.

2. Procédé selon la revendication 1, dans lequel l'enzyme oxydo-réductase est choisie dans le groupe constitué par laccase, peroxydase et un mélange de celles-ci.

3. Procédé selon la revendication 1 ou la revendication 2 dans lequel l'enzyme oxydo-réductase est immobilisée sur le substrat de support au moyen d'une liaison covalente.

4. Procédé selon l'une quelconque revendication précédente dans lequel le substrat de support est choisi dans le groupe constitué par tissus, matériaux non-tissés et matières plastiques.

5. Procédé selon l'une quelconque revendication précédente dans lequel le substrat de support est un corps creux tridimensionnel dans lequel la peroxydase est immobilisée sur la surface intérieure du corps creux.

6. Procédé selon l'une quelconque revendication précédente dans lequel le substrat de support est une particule inorganique.

7. Procédé selon l'une quelconque revendication précédente dans lequel la composition de nettoyage comprend de 0,01 % à 15 % en poids de la composition d'une source de peroxygène et l'enzyme oxydo-réductase est une peroxydase.

8. Procédé selon la revendication 7 dans lequel la source de peroxygène est choisie dans le groupe constitué par peroxyde d'hydrogène, un précurseur de peroxyde d'hydrogène, un système enzymatique de génération de peroxyde d'hydrogène, ou un acide peroxycarboxylique ou un sel de celui-ci et des mélanges de ceux-ci.

9. Procédé selon l'une quelconque revendication précédente dans lequel la lessive comprend un médiateur, choisi de préférence dans le groupe constitué par médiateurs à base organique, médiateurs à base de complexes de coordination de métal de transition et mélanges de ceux-ci.

10. Procédé selon la revendication 9 dans lequel le médiateur est immobilisé sur un substrat de support.

11. Procédé selon la revendication 9 ou la revendication 10 dans lequel l'enzyme oxydo-réductase est une peroxydase et le médiateur est un médiateur de peroxydase ayant la formule
Z1HN-NHZ2
dans lequel Z1, est l'un quelconque groupe organique par exemple, aromatique (substitué)-(hétéro)(polycyclique), (cyclo)-alkyle substitué contenant des hétéroatomes, et Z2 est un groupe de retrait d'électrons, choisi dans le groupe constitué par alkyl/(hétéro)aryl éventuellement substitué -sulfone, -sulfoxyde, - sulfonate, -carbonyle, -oxalyle, -amidoxalyle, 5 hydrazidoxalyle, -carboxyle et esters et sels de ceux-ci, amidyle, -hydrazidyle, nitrile.

12. Procédé selon la revendication 9 ou la revendication 10 dans lequel l'enzyme oxydo-réductase est une peroxydase et dans lequel le médiateur est un médiateur de peroxydase choisi dans le groupe constitué par acide phénoxazine-10-propionique, phénoxazine-10-hydroxyéthyle, phénothiazine-10-éthyl-4-carboxy, acide phénothiazine-10-propionique, chlorhydrate de promazine, alcool phénothiazine-10-éthylique et un mélange de ceux-ci.

13. Procédé selon l'une quelconque revendication précédente dans lequel l'enzyme oxydo-réductase comprend une enzyme laccase, de préférence la lessive comprend en outre un médiateur de laccase choisi dans le groupe constitué par 2,2'-azinobis-(3-éthylbenzthiazoline-6-sulfonate), 1-hydroxybenzotriazole, acide violurique, N-hydroxyacétanilide, syringate de méthyle, acétosyringone, syringaldézine, syringate de butyle, syringate de pentyle, syringate d'hexyle, syringate d'heptyle, alcool vanillylique, acide synapique, acétovanillone, et mélanges de ceux-ci.

14. Procédé selon l'une quelconque revendication précédente dans lequel la composition de nettoyage comprend un système tensioactif comprenant un agent tensioactif anionique et facultativement un agent tensioactif non ionique.

15. Procédé selon l'une quelconque revendication précédente dans lequel la surface comprend un tissu et le procédé est destiné au lessivage de tissus et dans lequel les tissus comprennent des tissus de couleurs mélangées, et de préférence à la suite de l'étape de lavage dans laquelle la surface est nettoyée par mise en contact de la surface avec la lessive, le substrat de support et l'enzyme oxydo-réductase sont séparés de la lessive et réutilisés dans une seconde étape de lavage permettant de nettoyer une seconde surface.
